# EUROPEAN PATENT SPECIFICATION

(11) **EP 2 893 344 B1**
(45) Date of publication and mention of the grant of the patent: **03.07.2019**
(21) Application number: 13835702.5
(22) Date of filing: 10.09.2013
(51) Int. Cl.: G01N 33/483, C12Q 1/37

(54) **SAME-DAY BLOOD CULTURE WITH DIGITAL MICROSCOPY**
TAGGLEICHE BLUTKULTUR MIT DIGITALER MIKROSKOPIE
CULTURE DE SANG DANS LA MÊME JOURNÉE AVEC MICROSCOPIE NUMÉRIQUE

(30) Priority: 10.09.2012 US 201261699191 P
(43) Date of publication of application: 15.07.2015
(73) Proprietor: Accelerate Diagnostics, Inc., Tucson, AZ 85714 (US)
(72) Inventor: METZGER, Steven W., Tucson, Arizona 85766 (US); HANCE, Kenneth Robert, Tucson, Arizona 85745 (US); HOWSON, David C., Denver, Colorado 80206 (US)
(74) Representative: Arends, William Gerrit
(86) International application number: PCT/US2013/059104
(87) International publication number: WO 2014/040088

(56) References cited:
- WO-A1-93/13197
- WO-A1-2010/062350
- WO-A1-2010/062352
- WO-A2-2006/015374
- US-B1- 6 242 188
- US-B2- 7 341 841
- M OHEIM: "High-throughput microscopy must re-invent the microscope rather than speed up its functions", BRITISH JOURNAL OF PHARMACOLOGY, vol. 152, no. 1, 1 September 2007 (2007-09-01), pages 1-4, XP055198062, ISSN: 0007-1188, DOI: 10.1038/sj.bjp.0707348
- MOLIN ET AL.: 'Rapid Detection of Bacterial Growth in Blood Cultures by Bioluminescent Assay of Bacterial ATP.' JOURNAL OF CLINICAL MICROBIOLOGY. September 1983, pages 521 - 525, XP055198061
- OHEIM.: 'High-throughput microscopy must re-invent the microscope rather than speed up its functions.' BR J PHARMACOL. vol. 152, no. 1, 02 July 2007, pages 1 - 4, XP055198062
- Metzger ET AL: "Rapid Identification of Resistance Phenotypes in Gram-Negative Bacilli Using Automated Digital Microscopy", Accelerate Diagnostics.com, 17 May 2009 (2009-05-17), pages C207-C207, XP055286127, Retrieved from the Internet: URL:http://acceleratediagnostics.com/docs/ ASM_2009_GN-a.pdf [retrieved on 2016-07-05]
- Yaseelan Palarasah ET AL: "Sodium Polyanethole Sulfonate as an Inhibitor of Activation of Complement Function in Blood Culture Systems", JOURNAL OF CLINICAL MICROBIOLOGY, vol. 48, no. 3, 1 March 2010 (2010-03-01), pages 908-914, XP055413378, US ISSN: 0095-1137, DOI: 10.1128/JCM.01985-09
- S Metzger ET AL: "3-Hour ESBL Detection from Positive Blood Cultures Using Multiplexed Automated Digital Microscopy (MADM)", Acceleratediagnostics.com, 16 June 2012 (2012-06-16), pages 1-1, XP055286118, Retrieved from the Internet: URL:http://acceleratediagnostics.com/docs/ ASM_2012_ESBL.pdf [retrieved on 2016-07-05]
- S Metzger ET AL: "Same-Day Blood Culture with Digital Microscopy", Accelerate Diagnostics.com, 9 September 2012 (2012-09-09), pages D1410-D1410, XP055286135, Retrieved from the Internet: URL:http://acceleratediagnostics.com/docs/ ICAAC_2012.pdf [retrieved on 2016-07-05]

## Description

### FIELD

The present disclosure relates generally to methods of rapidly culturing microorganisms from samples to facilitate rapid microorganism detection. More particularly, the disclosure relates to methods for growing the microorganism in the blood culture sample to increase the number of microorganisms available for detection while reducing the concentration of material in the sample that may interfere with downstream detection of the microorganisms in the sample.

### BACKGROUND

Critically ill patients who acquire a bloodstream infection must begin adequate antibiotic therapy as quickly as possible. The requirement for overnight culture creates an unacceptable delay. Rapid culture methods combined with detection methods such as automated digital microscopy offer the potential for same-day turnaround, including organism detection, identification, and drug resistance phenotype characterization, directly from a clinical specimen such as a blood sample.

Bacteremia due to multiple drug resistant organisms (MDRO) is increasing in frequency and growing in complexity. For critically ill patients, resistance can render initial therapy ineffective, delaying the start of effective antimicrobial therapy. Delay also prolongs exposure to broad-spectrum empiric therapy, creating selective pressure favoring emergent resistance. WO2010062352 discloses a method comprising mixing a blood sample with a combination of a detergent, such as saponin, enzymes, such as proteinases, and further including additional agents, such as reducing agents (e.g. 2-mercaptoethanol or DTT), buffering agents, and lytic agents. The compounds are added after cell growth.

Rapid culture methods disclosed herein have the potential to reduce turnaround time by facilitating analysis of live bacteria directly from a clinical specimen, eliminating the need for preliminary or preparative culture steps such as production of colony isolates or a positive blood culture.

### SUMMARY

The present invention relates to a method comprising the steps of:
a) introducing a culture medium, a lytic agent and a cellular debris-cleaving enzyme to a blood sample comprising erythrocytes and at least one live microbial cell, wherein the introduction of the lytic agent and the cellular debris-cleaving enzyme to the blood sample degrades the erythrocytes to produce a concentration of free heme in the blood sample capable of depressing growth of the at least one live microbial cell, wherein the blood sample is a direct-from-patient blood culture or a positive blood culture;
b) introducing a heme polymerase to the blood sample;
c) incubating the blood sample with the lytic agent and the cellular debris-cleaving enzyme and the heme polymerase for a period of time to produce a digested blood sample;
d) producing growth of the at least one live microbial cell during the period of time to yield a threshold number of live microbial cells required for identification; and
e) determining the species and/or strain of at least one of the live microbial cells using automated digital microscopy.

The present disclosure relates generally to methods for rapid culture of microorganisms in a sample, and more specifically, to methods for growth and/or recovery of live microbial cells directly from a sample. As set forth in more detail below, various disclosures herein provide advancements over prior art methods, particularly with regard to producing growth of microorganisms present in a sample along with a reduction in sample debris that may interfere with microorganism detection and toxic effects that may inhibit microorganism growth. The disclosure herein includes methods for selectively degrading non-viable microbial cells in a sample during a growth period for detection of viable microbial cells following the growth period.

In one aspect, the disclosure herein includes methods of obtaining a clinical specimen having a blood sample with a microbial cell, diluting a portion of the clinical specimen with a culture medium to produce a diluted specimen, subjecting the diluted specimen to a growth period to produce a growth culture, and detecting the microbial cell from the growth culture. In various parts of the disclosure, the clinical specimen is a direct-from-patient blood culture or a positive blood culture. In various further parts of the disclosure, a colony isolate is produced from the clinical specimen, and/or the method is applied directly to the clinical specimen. In a further part of the disclosure, the method also provides concentrating the growth culture. Various concentrating techniques are contemplated including centrifugally concentrating the growth culture having the microbial cell, and/or introducing an aliquot of the growth culture to a flowcell channel and electrokinetically concentrating the microbial cell onto a surface of the flowcell channel. In yet a further embodiment, the detecting step is performed using a multiplexed automated digital microscopy. In a further part of the disclosure, the detecting step includes one or more of detecting clonal growth associated with the microbial cell, determining an identity of the microbial cell, and determining antimicrobial resistance phenotype of the microbial cell. Two (2) or more antimicrobial resistance phenotypes may be determined. The disclosure herein includes a method which is performed in less than eight (8) hours, and the blood sample includes less than about 10 CFU/mL.

Also disclosed herein are methods of obtaining a clinical specimen having a microbial cell; introducing to the clinical specimen one or more of a culture medium, a lytic agent at a lytic agent concentration, and a debris-cleaving enzyme at an enzyme concentration; and incubating the sample for a first period of time to produce a digested sample having sample debris. The first period of time includes a minimum time to produce growth of the microbial cell to yield at least a threshold number of microbial cells for detection by a detection apparatus. The threshold number of microbial cells may be directly proportional to the sample debris concentration and/or the sample debris particle size. The desired concentration of debris-cleaving enzyme may be that which produces a reduction in at least one of the sample debris concentration and/or the sample debris particle size, and, optionally, at a rate that reduces interference with detection of microbial cells within the first period of time. The concentration of debris-cleaving enzyme preferably does not produce a condition of a depressed microbial cell growth rate in the first period of time. In various parts of the disclosure, the debris-cleaving enzyme may include a hydrolase, such as a protease, a peptidase, a nuclease, a lipase, an amylase, a glycosidase, a glycanase, and a hyaluronidase.

Also disclosed herein is a method of obtaining a blood sample; introducing a lytic agent and a protease to the blood sample, where the blood sample contains erythrocytes and a live microbial cell; introducing a detoxification agent to the blood sample; and incubating the blood sample for a first period of time to produce a digested blood sample. , The method may include the step of introducing a detoxification agent to the blood sample. The detoxification agent may aid in preventing a depression in a growth rate of the live microbial cell and/or producing an increase in the rate of growth rate. In various further parts of the disclosure, the lytic agent and/or protease degrade erythrocytes to produce a concentration of free heme in the sample. The concentration of free heme may depress the growth rate of the live microbial cell. In various further parts of the disclosure, a heme detoxification agent is introduced to the blood sample, including one or more of a heme polymerase, an antioxidant, a free radical scavenger, a reducing agent, a buffering agent, and agent that inactivates one or more antibiotics. The agent may increase the rate of beta-hematin formation, heme aggregate formation and/or oligomeric heme aggregate formation.

Another part of the disclosure relates to obtaining a clinical sample, not limited to a blood sample; performing a protease digestion of microbial cells within the sample; incubating the sample for a first period of time; and detecting the presence of a live target microbial cell. The protease digestion is suitable to selectively degrade non-viable target microbial cells, such as cellular and non-cellular debris, including blood sample debris, in a first period of time.

Another part of the disclosure relates to a method of obtaining a blood sample, and introducing a lytic agent and a debris-cleaving enzyme, such as a protease, to the blood sample. The blood sample may contain viable microbial cells and non-viable microbial cells. The lytic agent lyses the blood cells producing blood cell debris. The method further includes incubating the blood sample for a first period of time, such that the debris-cleaving enzyme may degrade one or more of the blood cell debris, non-viable microbial cells, and/or non-target cells. The method then further includes detecting the presence of viable microbial cells.

Another part of the disclosure relates to a method of preparing a clinical specimen to produce a sample; introducing a portion of the sample to an automated microscopy system which includes one or more of a microscope, a pipetting robot, a multichannel fluidic cassette, and an analyzer; detecting the presence of a live microorganism in the clinical specimen; and determining the identity of the live microorganism in the clinical specimen. The step of detecting the presence of a live microorganism may include detecting the presence of a growing clone. The identity of the live microorganism may include analysis of at least forty (40) growing clones. In yet another part of the disclsoure , the method further comprises determining a drug resistance phenotype of the live microorganism by the automated microscopy system.

Further areas of applicability will become apparent from the detailed description provided herein. It should be understood that the description and specific examples are intended for purposes of illustration only and are not intended to limit the scope of the present invention as defined in the claims.

### BRIEF DESCRIPTION OF THE DRAWINGS

The subject matter of the present disclosure is particularly pointed out and distinctly claimed in the concluding portion of the specification. A more complete understanding of the present invention, however, may best be obtained by referring to the detailed description when considered in connection with the drawing figures, wherein like numerals denote like elements and wherein:
FIG. 1A is a perspective view of a 32-channel disposable cassette in accordance with various embodiments;
FIG. 1B is a top view of an individual flowcell of a cassette in accordance with various embodiments;
FIG. 2 illustrates a process flow for rapid culture of blood samples followed by MADM analysis;
FIG. 3 shows examples of dark-field images for 3 hours of clone growth for SA without drug, SA in 6 µg/mL FOX, and for a Gram-negative rod (*E. coli*) without drug for morphology;
FIG. 4A-1 shows a darkfield image from an undigested sample;

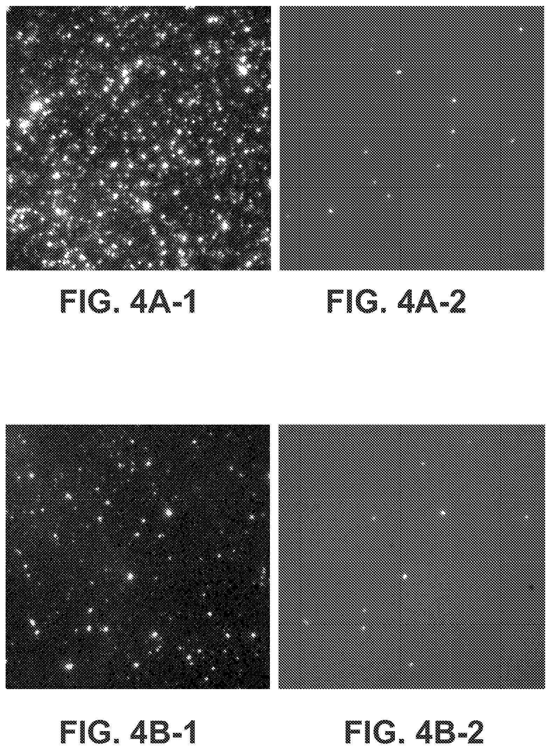

FIG. 4A-2 shows a darkfield image from a protease-digested sample;
FIG. 4B-1 shows a fluorescent image from an undigested sample;
FIG. 4B-2 shows a fluorescent image from a protease-digested sample;

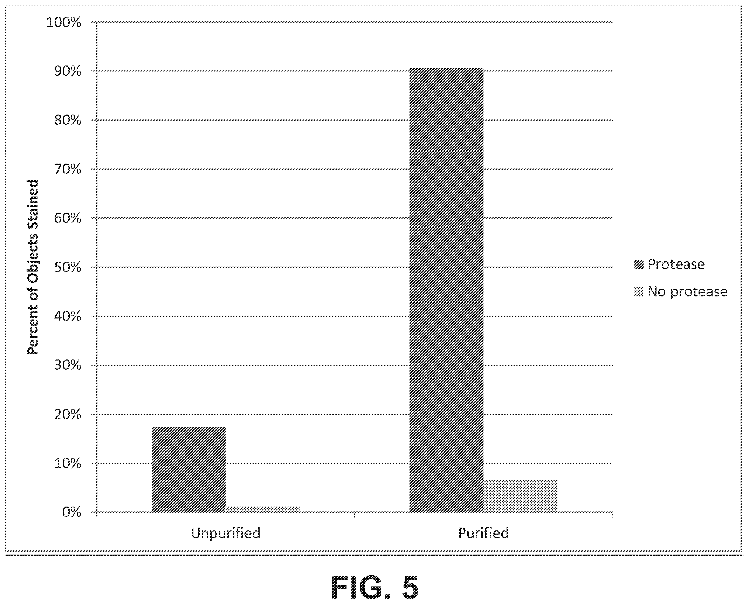

FIG. 5 shows the relative percentage of total darkfield objects detected as *E. coli* by FISH assay in the fluorescent image;

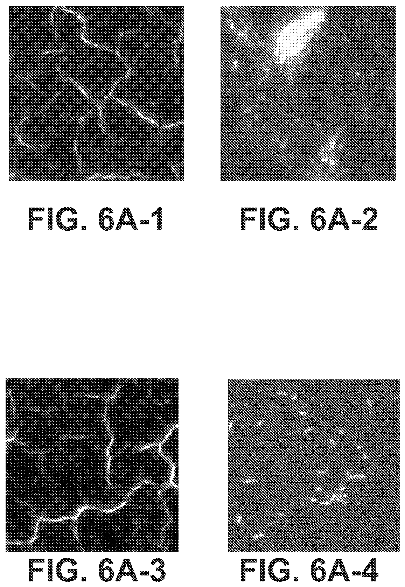

FIG. 6A-1 shows a darkfield image of an undigested, unpurified sample;
FIG. 6A-2 shows a darkfield image of an undigested, purified sample;
FIG. 6A-3 shows a darkfield image of a digested, unpurified sample;
FIG. 6A-4 shows a darkfield image of a digested, purified sample;

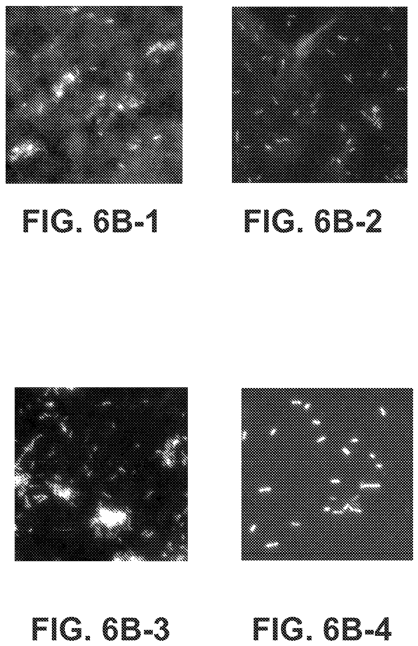

FIG. 6B-1 shows a fluorescent image of an undigested, unpurified sample;
FIG. 6B-2 shows a fluorescent image of an undigested, purified sample;
FIG. 6B-3 shows a fluorescent image of a digested, unpurified sample;
FIG. 6B-4 shows a fluorescent image of a digested, purified sample;

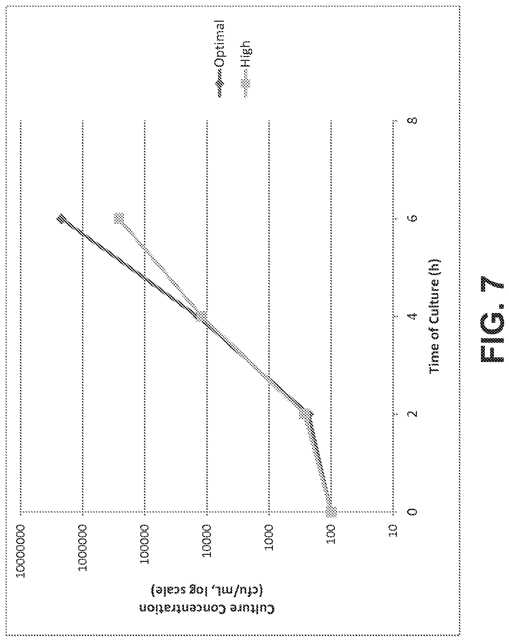

FIG. 7 is a graph showing an increase in bacteria for the optimal and high protease conditions at each of the time points;

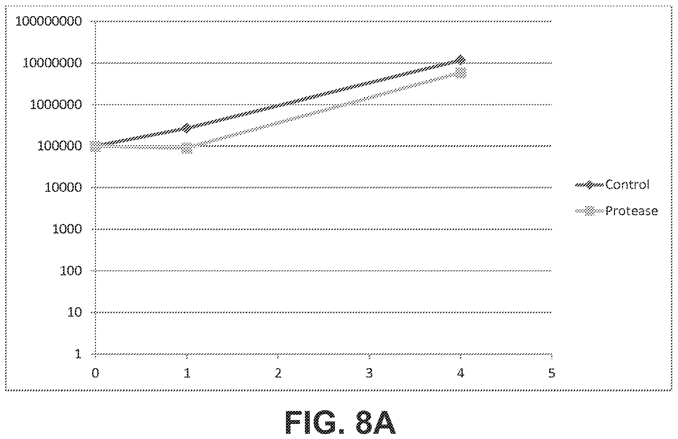

FIG. 8A is a graph showing the concentration of bacteria detected in a FISH assay at 1 and 4 hours versus the known concentration spiked at the beginning of the assay for the *P. aeruginosa* growth control conditions with and without protease;

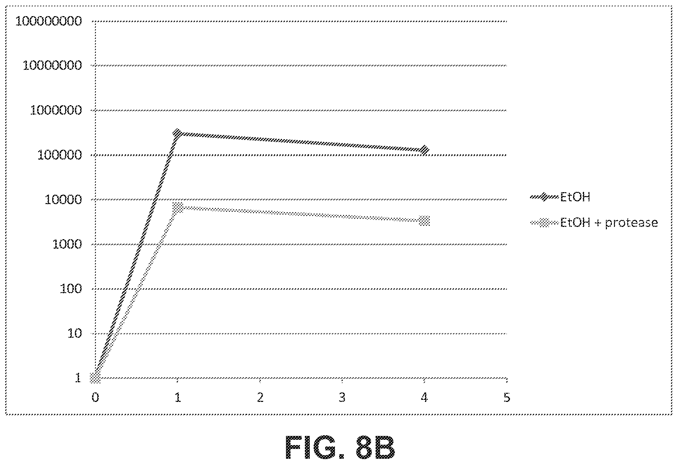

FIG. 8B is a graph showing the concentration of bacteria detected in a FISH assay at 1 and 4 hours versus the known concentration spiked at the beginning of the assay for the *P. aeruginosa* ethanol-treated conditions with and without protease; and

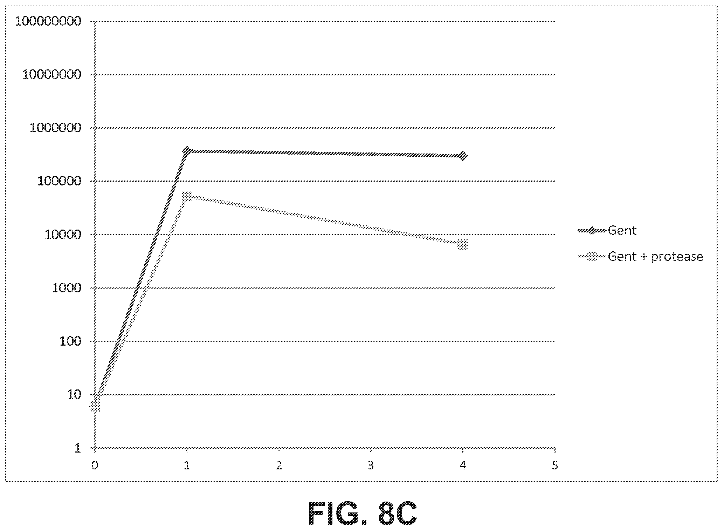

FIG. 8C is a graph showing the concentration of bacteria detected in the FISH assay at 1 and 4 hours versus the known concentration spiked at the beginning of the assay for the *P. aeruginosa* gentamycin-treated conditions with and without protease.

### DETAILED DESCRIPTION

The following description is merely exemplary in nature and is not intended to limit the present invention, its applications, or its uses. It should be understood that throughout the drawings, corresponding reference numerals indicate like or corresponding parts and features. The description of specific examples indicated in various embodiments of the present invention are intended for purposes of illustration only and are not intended to limit the scope of the invention disclosed herein. Moreover, recitation of multiple embodiments having stated features is not intended to exclude other embodiments having additional features or other embodiments incorporating different combinations of the stated features.

Furthermore, the detailed description of various embodiments herein makes reference to the accompanying drawing figures, which show various embodiments by way of illustration. While the embodiments are described in sufficient detail to enable those skilled in the art to practice the invention, it should be understood that other embodiments may be realized and that logical and mechanical changes may be made without departing from the spirit and scope of the present invention. Thus, the detailed description herein is presented for purposes of illustration only and not of limitation. For example, steps or functions recited in descriptions any method, system, or process, may be executed in any order and are not limited to the order presented. Moreover, any of the step or functions thereof may be outsourced to or performed by one or more third parties. Furthermore, any reference to singular includes plural embodiments, and any reference to more than one component may include a singular embodiment

The present disclosure relates generally to methods for rapid culture of microorganisms in a sample, and more specifically, to methods for growth and/or recovery of live microbial cells directly from a sample. As set forth in more detail below, various embodiments provide advancements over prior art methods, particularly with regard to producing growth of microorganisms present in a sample along with a reduction in sample debris that may interfere with microorganism detection and toxic effects that may inhibit microorganism growth.

### Sample

The present disclosure provides methods of detecting a microorganism present in a clinical specimen or sample. As used herein, the terms "clinical specimen" and "sample" may be used interchangeably. In accordance with various embodiments, a clinical specimen may comprise a sample from any number of sources, such as bodily fluids (including, but not limited to, blood, urine, serum, lymph, saliva, anal and vaginal secretions, perspiration, peritoneal fluid, pleural fluid, effusions, ascites, purulent secretions, lavage fluids, drained fluids, brush cytology specimens, biopsy tissue, explanted medical devices, infected catheters, pus, biofilms, and semen) of virtually any organism. While the methods disclosed herein may be of particular relevance to samples obtained from a biological source, other sample types such as environmental samples (including, but not limited to, air, agricultural, water, and soil samples) may also be used in the methods of the present disclosure. Likewise, samples can be taken from food processing, which can include both input samples (e.g., grains, milk, or animal carcasses), samples in intermediate steps of processing, as well as finished food ready for the consumer. The value of the methods disclosed herein for veterinary applications should be appreciated as well, for example, with respect to its use for the analysis of milk in the diagnosis and treatment of mastitis, or the analysis of respiratory samples for the diagnosis of bovine respiratory disease.

### Growth

As used herein, the term "growth" may include any measurable change attributable to or occurring within the life history of an organism, whether that change occurs under static external conditions or in response to a change in an internal or external event or condition. "Growth" can be used to refer to one or more changes associated with a single microorganism, or growth can be used to refer to a net or collective change in a group, collection, or population of organisms, whether derived from a single parental cell or from multiple parental cells. "Growth" in the case of microorganisms that are cells (e.g., bacteria, protozoa, and fungi) can refer to an increase in an attribute of a microorganism, including such attributes as mass, cell number, cell metabolism products, or any other experimentally observable attribute of a microorganism.

### Culture medium

As used herein, the term "culture medium" is used to refer to a liquid or a gel medium used to support the growth of microorganisms. For example, a culture medium may comprise a nutrient broth or a liquid nutrient medium. Gel or solid media may also be used as a culture medium.

### Lytic agent

As used herein, the term "lytic agent" is used to describe any chemical compound or formulation that may be used to cause or contribute to lysis of a cell. In accordance with various embodiments, introduction of and/or incubation of a lytic agent with a biological sample or clinical specimen can produce lysis and/or disruption of cells present in the sample. In accordance with various embodiments and as used herein, introducing a lytic agent into a biological sample, such as a blood sample, may result in the lysis of eukaryotic cells present in the biological sample, including, for example, erythrocytes (i.e., red blood cells), leukocytes (i.e., white blood cells, including agranulocyte and granulocyte forms such as neutrophils, eosinophils, basophils, lymphocytes, monocytes, and macrophages), thrombocytes (i.e., platelets) and the like. Introduction of a lytic agent to samples obtained from other sources may likewise disrupt various other cell types that may be present in a sample. In accordance with various embodiments, a lytic agent may be used that selectively produces lysis of eukaryotic cells present in a particular biological sample type without lysing or otherwise inhibiting the growth of prokaryotic cells such as bacterial cells that may also be present in the sample. Lysis of cells in a sample may produce sample debris such as cell membrane fragments and other cellular components, as explained in more detail below. In various embodiments, a lytic agent may also contribute to disruption, dissolution, and/or degradation of cellular components and sample debris that may be present in a sample following cell lysis.

In accordance with various embodiments, a lytic agent may comprise a detergent. In various embodiments, a detergent may be a non-ionic or a zwitterionic detergent. Ionic detergents may also be used in accordance with various embodiments of the present disclosure. Examples of non-ionic detergents that may be used as a lytic agent accordance with various embodiments of the present disclosure include saponin, Tween-20, Triton X-100, NP-40, and the like. In accordance with various embodiments, a lytic agent and/or a lytic agent concentration that is non-toxic to a microbial cell may be selected for treatment of a biological sample. In other embodiments, a lytic agent and/or a lytic agent concentration may be selected that provides effective lysis and/or sample debris clearance for a biological sample while producing a minimal inhibition of growth of a microbial cell in the sample. For example, a lytic agent and/or lytic agent concentration may be selected for a particular sample type and a target microbial cell species based on predetermined compatibility of the lytic agent and/or lytic agent concentration with the growth of the target microbial cell species and the lysis of the eukaryotic cell types present in the sample type.

### Debris cleavage

In accordance with various embodiments of the present disclosure, a sample may comprise sample debris. As used herein, the term "sample debris" may be used to refer to non-microbial cells, cell fragments, cell components, and any other cellular or non-cellular particular or macromolecular material that may be present in a sample. In accordance with various embodiments, sample debris may be present in a sample or may be produced as a result of sample handling or processing during various steps of the methods disclosed herein. For example, introduction of a lytic agent to a blood sample may produce lysis of blood cells present in the sample, and the cell membrane and cellular contents may comprise sample debris. As used herein, sample debris may also comprise intact or unlysed non-microbial cells, as well as non-viable microbial cells or cell components.

In accordance with various embodiments of the present disclosure, the presence of sample debris may interfere with an ability to recover and/or detect microbial cells in the sample. In various embodiments and as explained in greater detail below, one or more enzymes, referred to herein as "debris-cleaving enzymes," may be introduced to a sample to cleave or degrade sample debris. Sample debris cleavage (also referred to herein as "digestion") may reduce a sample debris concentration and/or a sample debris particle size in accordance with various embodiments of the present disclosure.

In accordance with various embodiments, a debris-cleaving enzyme may comprise an enzyme classified in Enzyme Commission group 3 (EC 3), which includes hydrolases that catalyze the formation of two products from a substrate by hydrolysis. In various embodiments, a hydrolase used as a debris-cleaving enzyme may comprise, for example, a protease, a peptidase, a nuclease, an amylase, a glycosidase, a glycanase, or a hyaluronidase. Combinations of different debris-cleaving enzymes, such as proteases with different recognition sites, or a combination of a protease and a nuclease, may be used to perform debris cleavage in accordance with various embodiments of the present disclosure. In various embodiments, a protease or a peptidase may comprise an exopeptidase or an endopeptidase. Examples of proteases that may be used in accordance with various embodiments include Pronase and Rhozyme. Other hydrolases that may be used include esterases such as lipases (including phospholipase) and nucleases (including exonucleases and endonucleases). In various embodiments, nucleases may be used to decrease a viscosity of respiratory samples. Still other hydrolases may include glycoside hydrolases such as endoglycosidases, exoglycosidases, glycanases, amylases, and hyaluronidases. In various embodiments, glycoside hydrolases may be used to degrade mucin in respiratory samples. In addition to hydrolases, other categories of enzymes catalyzing other categories of intramolecular bond-cleaving reactions, such as lyases (EC 4), may also be used in accordance with various embodiments of the present disclosure.

In various embodiments, a chemical agent may be used to provide a debris-cleaving function instead of or in addition to a debris-cleaving enzyme. In accordance with various embodiments, various chemical agents may be used to cleave intramolecular bonds and produce sample debris digestion. For example, cyanogen bromide, BNPS-skatole, formic acid, hydroxylamine, and 2-nitro-5-thiocyanobenzoic acid may be used to cleave peptide bonds. Any chemical agent that may be used to reduce a concentration and/or a particle size of sample debris may be used as a debris-cleaving agent in accordance with various embodiments of the present disclosure.

In accordance with various embodiments, degradation, cleavage, or any other reduction in the particle size sample debris to smaller fragments or constituent molecules may produce products that may serve as additional nutrients that may promote the growth of a microorganism.

### Toxic Effects and Detoxification Agents

While not wishing to be bound by theory, it is believed that culturing and/or growth of microorganisms under various conditions may produce chemical or other conditions in a sample that can inhibit microorganism growth. Likewise, treatment of a sample, such as sample digestion by lytic agent treatment and/or debris-cleavage may produce also produce chemical or other conditions in the sample that can inhibit microorganism growth. As used herein, a chemical or other condition that can inhibit microorganism growth (i.e., produce a depression in the growth rate of a microorganism) may be variously referred to as a toxin, a toxic condition, or a toxic effect. Similarly, as used herein, a detoxification agent may be any chemical, enzymatic, or other treatment that may reduce a toxic condition, reduce or prevent a depression in the growth rate of a microorganism, or produce an increase in a growth rate of a microorganism.

In accordance with various embodiments, growth of microorganisms in a culture medium comprising a blood sample, as well as other manipulations, such as lysis and/or proteolytic digestion of a blood sample or blood sample components in a culture medium, may produce chemical compounds or effects that inhibit bacterial growth. Similarly, chemical compounds that can inhibit bacterial growth may be present in a blood sample or produced by a blood sample. For example free radical compounds, acidifying compounds, free heme, antibiotics, host immune factors, and the like, may be present in a blood sample, produced during digestion of a sample comprising a blood sample, or produced during growth of a microorganism in a culture medium comprising a blood sample.

In accordance with various embodiments, a sample may comprise host immune factors. Host immune factors may include cellular and humoral immune components such as complement proteins, antibodies, and leukocytes that may inhibit microbial growth. The effects of host immune factors on microbial growth may be reduced by treatment of the sample with detoxification agents such as protectants, lytic agents, and proteases. For example, sodium polyanethol sulfonate (SPS; Liquoid, Hoffman-La Roche) is a synthetic compound that is a protectant and a common additive in blood culture media due to is anticoagulant, anticomplement, and antiphagocytic properties. In accordance with various embodiments, SPS may serve to enhance the rate and speed of bacterial growth in a blood sample-derived culture by counteracting certain bacterial inhibitors found in human blood.

In various embodiments, antibiotics present in the bloodstream of a patient undergoing antibiotic treatment may be present in a blood sample in a concentration sufficient to inhibit bacterial growth without fully killing the bacteria. In accordance with various embodiments, a blood sample and/or culture medium comprising a blood sample may be treated with a detoxifying agent that inactivates the antibiotic, such as activated charcoal, ion exchange resins, or other agents that bind or otherwise neutralize the antibiotic.

In accordance with various embodiments, lysis of blood cells may result in acidification of a culture medium, such as by a release of protons from leukocyte lysosomes. Acidification of a culture medium may inhibit the growth of various bacteria, such as *Streptococcus pneumoniae.* In various embodiments, a buffering agent may be added to a culture medium to maintain a pH compatible with achieving an optimal growth rate of a microorganism. For example, Tris buffer may be added to a culture medium at a concentration of from 1 mM to 10 mM to facilitate maintenance of a stable pH during a sample preparation and/or bacterial growth step.

In accordance with various embodiments, reactive oxygen species and other free radical compounds may be produced during a growth period and/or digestion treatment. In various embodiments, anti-oxidant compounds and/or free radical scavenging compounds may be introduced to a culture medium to reduce a toxic effect caused by the presence of oxidants and free-radicals. For example, ascorbic acid, carotenoids such as beta-carotene, and tocopherols such as alpha-tocopherol, may be added to a sample during a growth or digestion period to reduce a toxic effect of reactive oxygen species, free radical compounds, or other oxidants that may be present in a sample. In various embodiments, an antioxidant compound may be added at a concentration of 10 µM to 1 mM. In accordance with various embodiments, a detoxification agent may be an antioxidant, a reducing agent, or a free radical scavenger. In various embodiments, a free radical scavenger may be an alkyl peroxyl radical scavenger.

### Overdigestion

While not wishing to be bound by theory, it is believed that protease digestion of blood culture components can produce digestion products that negatively affect the growth of microorganism. In particular, it is believed that the methods disclosed herein can produce high concentrations of free heme as a result of the blood sample lysis and protease digestion process. Free heme may result from lysis of erythrocytes and digestion of hemoglobin, releasing free heme. Increased concentrations of free heme in a protease digested sample may likewise be a function of digestion and/or breakdown of other blood sample components that may serve to scavenge or otherwise interact with free heme, such as human serum albumin, hemopexin, serum lipocalin α₁-microglobulin, and the like.

While not wishing to be bound the theory, it is believed that the presence of free heme in the lysed and/or digested blood cultures may have a toxic effect on, or may otherwise inhibit or have a depressive effect on, the growth rate of certain microorganisms that may be present in the blood culture. While the mechanisms of heme toxicity are unknown and may vary with bacterial species, in accordance with various embodiments of the present disclosure, an increasing concentration of free heme that may result from lysis and/or digestion of blood culture components to an increasing level of completion by, for example, relatively long protease digestion periods, protease digestion with relatively high concentrations of protease, or the like, may negatively influence the growth rate of a microorganism present in the blood culture. In accordance with various embodiments, the growth rate of a microorganism present in a blood culture subject to protease digestion may demonstrate a depression or reduction that is dependent on and/or a function of protease treatment. In accordance with various embodiments, the effect of a protease-dependent reduction in growth rate may not influence the growth rate of a microorganism in a blood culture immediately at introduction of a protease to the blood culture comprising the microorganism. A protease dependent reduction in growth rate may begin to influence the growth rate of a microorganism in a blood culture after a period of time following introduction of the protease to the blood culture. The period of time from introduction of the protease to a time point at which a protease-dependent reduction in growth rate may begin to affect the growth rate of a microorganism may depend on the particular protease or proteases used to perform protease digestion, the concentration of the protease, the duration of protease digestion, the concentration of blood in the sample, individual variation in blood samples, the temperature at which protease digestion is performed, the presence and concentrations of other components such as detergents, protectants, reducing agents, and the like.

Similarly, the effect of protease digestion on the growth rate of a microorganism may depend on the species and/or strain of the microorganism. For example, assuming that protease digestion of a blood culture produces free heme, and that the presence of free heme in a blood culture may have a negative influence on the growth rate of various microorganisms, different microorganisms may have different sensitivities to free heme, such as different threshold concentrations at which free heme begins to exert a negative influence on microorganism growth or viability, as well as differences in a magnitude of response.

The enzyme concentration of the debris-cleaving enzyme may be adjusted to optimize a rate of sample debris degradation, including a rate of sample debris concentration reduction and/or a rate of sample debris particle size reduction, such that the enzyme concentration does not produce a condition of a depressed microbial cell growth rate.

In accordance with the present invention, a detoxification agent comprising a heme polymerase is added to a sample to reduce a toxic effect of free heme in a sample. For example, the detoxification agent increases a rate of heme aggregate formation or beta-hematin formation. In various embodiments, the heme polymerase may be protease resistant.

### Detection threshold

In accordance with various embodiments detection of microbial cells in a sample may be performed using a detection apparatus, system, method, or a combination thereof. In various embodiments, detection may require a threshold number of microbial cells. Incubation of a sample for a first period of time may be required to produce growth of a microbial cell in a sample to achieve at least a threshold number of microbial cells required for detection by a detection apparatus, system, or method. Similarly, in accordance with various embodiments, identification and antimicrobial susceptibility testing may also require a threshold number of microbial cells. The threshold number of microbial cells required for various downstream analyses may vary. Likewise, the known or anticipated starting number of microbial cells in a clinical specimen, the growth rate of a target microbial cell, and other factors may influence the minimum period of time necessary to produce a level of growth necessary to yield a threshold number of cells.

In accordance with various embodiments, the presence of sample debris in a sample may influence the threshold number of microbial cells required for detection. In various embodiments, at least one of a sample debris concentration and a sample debris particle size may influence the number of microbial cells required for detection. For example, the presence of a high concentration of sample debris and/or sample debris having a large particle size may interfere with the detection of microbial cells. In accordance with various embodiments, a threshold number of microbial cells required for detection may be directly proportional to at least one of a sample debris concentration and a sample debris particle size. For example, reduction of at least one of a sample debris concentration and a sample debris particle size, such as by digestion with a debris-cleaving enzyme, may reduce a threshold number of microbial cells required for detection. Similarly, reduction of a threshold number microbial cells required for detection due to digestion of sample debris may reduce the period of time required to produce growth the of a microbial cell to reach the detection threshold.

### EXAMPLE 1

### Methods

29 aliquots of 10 mL each were taken from two short-fill CPD blood bank bags. Each sample received isolate spikes to make nominal 5 CFU/mL of bacterial target species. These included 14 *Staphylococcus aureus* (SA), and 3 *Pseudomonas aeruginosa* (PA) plus 12 non-target Gram-negative bacilli. Each sample was diluted 4-fold to promote growth. 35 °C incubation for 4 hours was followed by centrifugation, with final resuspension in 1 mL of electrokinetic buffer. 16 flowcell channels in a multichannel fluidic cassette each received 20 µL of sample, followed by 5-minute electrokinetic concentration and surface capture. Liquid (40 °C) Mueller-Hinton agar with and without antimicrobials was then exchanged through each channel and gelled. Automated microscopy acquired images at 10-minute intervals for 3 hours. Image analysis detected clonal growth, identified SA and PA, and simultaneously performed resistance phenotype tests using 32 µg/mL amikacin, 8 µg/mL imipenem, 6 µg/mL cefoxitin, or 0.5 µg/mL clindamycin. Controls included quantitative culturing, disk diffusion tests for isolate resistance phenotype, and 20 blood samples without spikes.

### Results

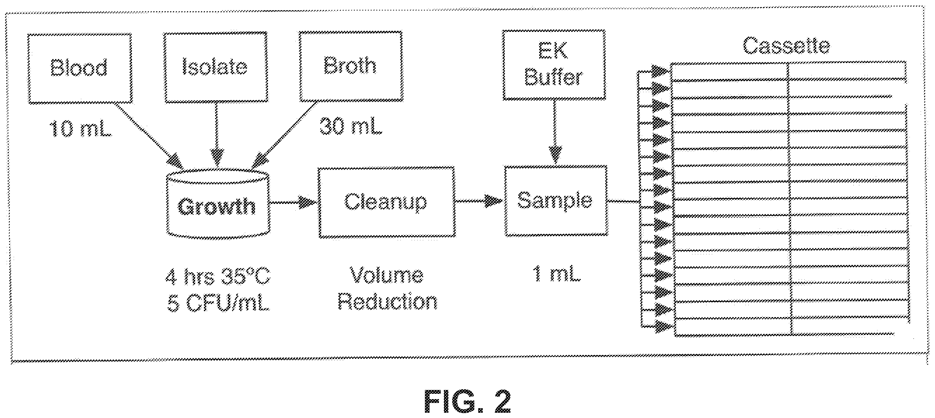

Simulated blood specimens consisted of isolates spiked into 10 mL each of 29 aliquots of banked blood to make approximately 5 CFU/mL, confirmed by quantitative culture. Spiked isolates included 14 *Staphylococcus aureus* (SA), 3 *Pseudomonas aeruginosa* (PA), or 12 non-target species. Dilution of each sample with 30 mL of modified TSB culture medium promoted growth. 20 additional control aliquots contained no spikes. 4-hour incubation at 35 °C, followed with brief spin cleanup, ended with pellet resuspension into an electrokinetic buffer to make 1 mL. 20 µL sample aliquots were then pipetted into 14 cassette flowcells (Fig. 2). A 5-minute low-voltage electrical field concentrated bacteria to the lower surface of each flowcell where a capture coating immobilized the bacterial cells.

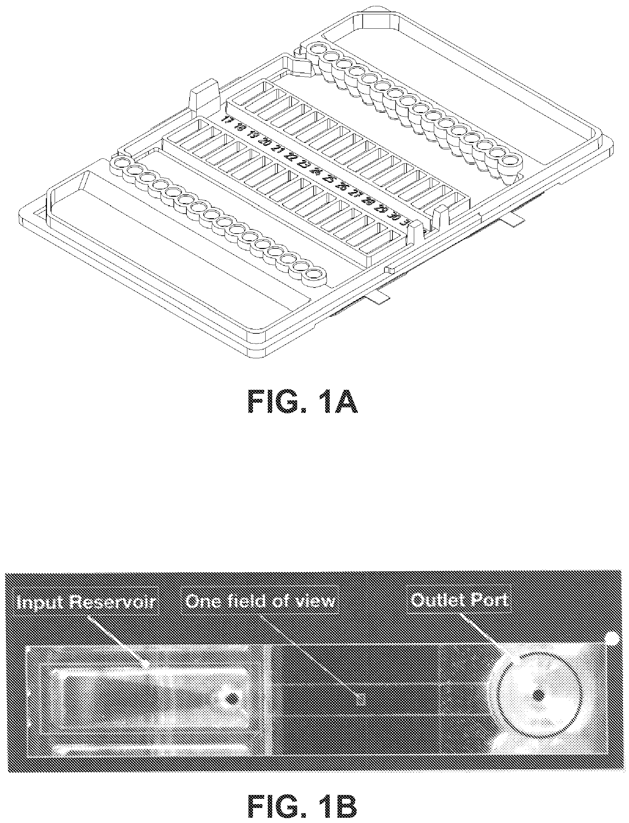

The MADM system used a custom microscope and pipetting robot, plus custom image analysis and experiment control software. 32-channel disposable cassettes (FIG. 1) enabled live microbial cell immobilization for microscopy and fluid exchanges for different test agents. The microscope scanned 40 image fields in each flowcell channel, each channel having organisms extracted from about 3.5 µL of prepared inoculum.

The system acquired dark-field images every 10 minutes. The analyzer applied identification algorithms to each individual immobilized cell that exhibited growth. 6 channels provided data for ID algorithms to score individual organisms and their progeny clones. ID variables included cell morphology, clone growth morphology, clone growth rate, and other factors. The analyzer computed ID probability based on the number of related clones and their scores.

Organism detection required ≥4 growing clones (GC). Recovery yielded SA GC counts that exceeded CFU as determined by culturing because of near-complete clump disruption in most samples. Counting combined results in multiple channels when appropriate. Identification required ≥40 GC, and each phenotype test required ≥40 GC. MADM detected growth in 29/29 spiked samples and no growth in 20/20 non-spiked controls. Growth sufficient for ID occurred in 23/29 samples. 4 SA samples clumped excessively, precluding ID scoring. 2 PA samples grew too slowly (<1.1 div/hr) to achieve 40 GC in the fixed 4-hour growth period (5 hours would suffice). SA growth rates were ≥1.5 div/hr. MADM identified 1/1 PA and 10/10 SA. One false PA ID occurred out of 22 non-target samples to yield 100% sensitivity and 97% specificity. The false ID was attributable to a known imaging aberration, later corrected.

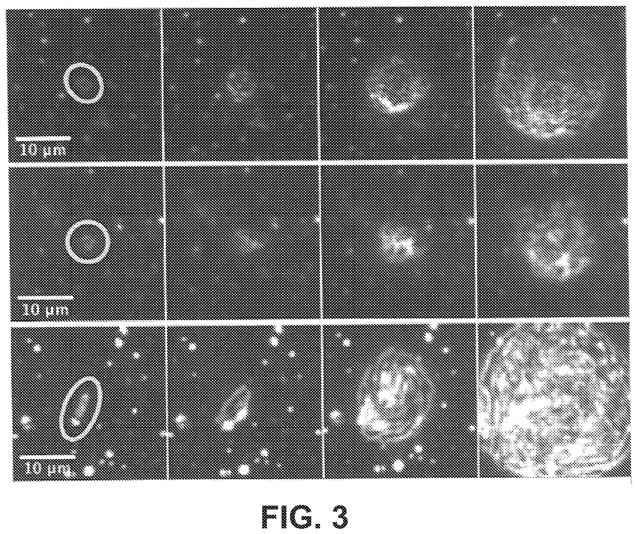

FIG. 3 shows examples of dark-field images for 3 hours of clone growth for SA without drug, SA in 6 µg/mL FOX, and for a Gram-negative rod (*E. coli*) without drug for morphology comparison.

MADM reported drug resistance in 19/20 adequate samples with one false MSSA, yielding 89% sensitivity and 100% specificity. Table 1 summarizes SA data for overall concordance with comparator results.

This pilot study asked whether major pathogens grow quickly enough to enable same-day diagnostic testing directly with bacteremic blood samples using microscopy. MADM had previously analyzed small numbers of live microbial cells extracted from other specimen types. This study demonstrated that 4 hours of growth in a common nutrient medium provides enough live clones for MADM analysis with fast-growing cells (>1.1 div/hour growth rate in the conditions tested). PA required slightly longer times for adequate testing, estimated at 5 hours. These results provide parameters for determining requirements for practical application. Given the number of GC required for a test (40 with the study prototype), number of tests, and the slowest target organism growth, straightforward calculation derives the minimum growth duration needed. Fastest possible turnaround time results from maximizing growth rate while minimizing the GC needed per test, and minimizing the required number of tests and their duration.

Within 8 hours starting with blood, automated microscopy successfully identified target pathogens and detected drug resistance phenotypes for a major species of live bacterial cells extracted directly from a small volume of simulated bacteremic blood. Diagnostic analysis using individual live-cell methods enables rapid turnaround without first requiring colony isolates. The probabilistic identification scoring achieved high concordance with clinical lab results. Resistance phenotype analysis also achieved high concordance. This analytical strategy can also use responses of individual clones to identify organism subpopulations and resistance phenotypes within polymicrobial specimens.

MADM enables diagnostic analysis of live microbial cells extracted after brief growth in culture medium.

**TABLE 1**

| Concordance of results for MADM and comparator results. | | | |
|---|---|---|---|
| **IDENTIFICATION (Adequate Growth N=23)** | | | |
| ***S. aureus*** | **True Neg** | **True Pos** | **Accuracy** |
| MADM-Pos | 0 | 10 | Sensitivity 100% (CI 66-100%) |
| MADM-Neg | 23 | 0 | Specificity 100% (CI 72-100%) |

| **PHENOTYPE: MRSA (Adequate Growth N=10)** | | | |
|---|---|---|---|
| ***S. aureus*** | **True Neg** | **True Pos** | **Accuracy** |
| MADM-Pos | 0 | 4 | Sensitivity 80% (CI 30-100%) |
| MADM-Neg | 5 | 1 | Specificity 100% (CI 46-100%) |

| **PHENOTYPE: CLI-R (Adequate Growth N=10)** | | | |
|---|---|---|---|
| ***S. aureus*** | **True Neg** | **True Pos** | **Accuracy** |
| MADM-Pos | 0 | 4 | Sensitivity 100% (CI 40-100%) |
| MADM-Neg | 6 | 0 | Specificity 100% (CI 52-100%) |

### EXAMPLE 2

### Effect of Protease Concentration Adjustment for Higher Cell Concentration Sample on Digestion on Sample Debris Concentration

### Introduction

Enzyme concentration can be optimized for shorter or longer digests to accommodate different requirements for specimen analysis. High bacteria concentration specimens similar to those taken from positive blood culture bottles can be processed with higher concentration of protease for a shorter period of time to enable rapid analysis. For such an analysis, minimal growth is required since the bacteria are in sufficient concentration without growth. However, some debris removal may be desired to allow viewing of live bacteria using time-lapse darkfield or other imaging techniques. This experiment illustrates a method for detecting the bacteria within the debris field using fluorescence in-situ hybridization, using comparison of darkfield and fluorescent images to determine which objects are bacteria.

### Methods

A mock blood culture specimen was created by addition of 10 mL of blood to a BioMerieux BacT/Alert Standard Aerobic bottle. A 1.0 mL portion of this specimen was used for each condition. Each sample was treated with saponin to a final concentration of 4 mg/mL and SPS to a final concentration of 0.96 mg/mL. Samples were then spiked with S. aureus bacteria to a final concentration of 1x106 cfu/mL. The samples were either treated with a solution containing 2 mg/mL (diluted in tryptic soy broth) or an equivalent volume of tryptic soy broth for undigested control. Samples were incubated at 35C with agitation for 1 hour. Each sample was processed to remove debris and exchange to the electrokinetic concentration buffer using centrifugation washing. Samples were added to wells of a microfluidic cassette having a conductive top and conductive slide and concentrated to and immobilized onto the poly-L-lysine coated slide surface by applying 1.5V for 5 minutes. The wells were washed with tris-buffered saline and then the cells were permeabilized for FISH staining using a combination of lysozyme and lysostaphin enzymes for 5 minutes followed by treatment with 80% ethanol for 5 minutes. Custom fluorochrome-labeled FISH probes directed at specific sequences in the 16S rRNA S. aureus were used to probe the permeabilized samples for 10 minutes using stringent conditions. The hybridization was followed by 2-5 minute washes with stringent wash buffer. The stained samples were imaged for fluorescence and darkfield debris analysis to visualize the bacteria within the debris field.

### Results

Table 2 shows the darkfield and fluorescent imaging results for each of the conditions. The data are presented as the percentage of the total darkfield objects detected as stained cells in the fluorescent image. The undigested sample contained a significant amount of debris that was equal in intensity and outnumbered the bacteria by more than 10 to 1. The digested sample contained fewer debris objects of equal intensity to the bacteria. Figures 4A-1, 4A-2, 4B-1, and 4B-2 show the darkfield and fluorescent images for these conditions showing the debris reduction resulting from the protease treatment. Images are 200 by 200 micron, cropped from full field of view images. The reduced sample debris concentration of the protease digested sample reduced interference with visualization of the bacteria in the darkfield imaging mode. This experiment demonstrates that, given a higher cell concentration, protease treatment can be effective at reducing debris in a short period of time using a higher enzyme concentration than that optimized for a 4 hour growth and digestion period that can be necessary to increase cell numbers to a detectable level from a lower cell concentration sample.

**TABLE 2**

| Comparison of microbial cell detection by FISH following protease digestion. | | |
|---|---|---|
| Condition | Total darkfield objects | *S. aureus* cells detected by FISH as a percentage of total objects detected |
| Protease-treated | 2393 | 4.9% |
| Untreated | 10254 | 2.0% |

### EXAMPLE 3

### Protease Digestion with Centrifugal Concentration of Bacteria and Conventional Slide-Based FISH Detection

Certain detection methods are not amenable to analysis of the specimen without further purification. Slide-based FISH staining is such an assay when the unpurified specimen is dried onto a slide as is typical with published methods on positive blood culture bottles. This example illustrates a method in which a single centrifugation step is sufficient to remove residual blood debris, purifying the bacteria for analysis, and allowing visualization of bacteria using only darkfield imaging.

A mock blood culture specimen was created by addition of 10 mL of blood to a BioMerieux BacT/Alert Standard Aerobic bottle. A 1.0 mL portion of this specimen was used for each condition. Each sample was treated with saponin to a final concentration of 4 mg/mL and SPS to a final concentration of 0.96 mg/mL. Samples were then spiked with *E*. *coli* bacteria to a final concentration of 1x10⁴ cfu/mL. The samples were either treated with a solution containing 0.2 mg/mL (diluted in tryptic soy broth) or an equivalent volume of tryptic soy broth for undigested control. Samples were incubated at 35C with agitation for 4 hours. A portion of each sample was removed for analysis of the unpurified samples. The remaining portion of each sample was centrifuged for 4 minutes at 8000xg and the supernatants were removed. The pellet containing debris and bacteria were resuspended with a volume of 1 mM histidine buffer to reach the pre-centrifugation volume. A 20 microliter portion of each unpurified and purified sample was dried onto a well of an 8-well silanized slide at 52C. The slide was processed for FISH staining using specific *E. coli* FISH probes. Briefly, *E. coli* were permeabilized by treatment with 80% ethanol for 5 minutes and then dried. Custom fluorochrome-labeled FISH probes directed at specific sequences in the 16S rRNA of *E. coli* were used to probe the permeabilized samples for 10 minutes using stringent conditions. The hybridization was followed a single 10 minute wash with stringent wash buffer.

For analysis, the slide was dried and then mounted for fluorescent imaging. The images were taken using an automated microscope that first imaged darkfield and then fluorescence imaging was performed on the site. Images were collected for the following conditions:
- Undigested, unpurified;
- Undigested, purified by one centrifugation wash;
- Digested, unpurified; and
- Digested, purified by one centrifugation wash.

The images were analyzed using an automated particle detection algorithm that seeks objects similar in size and brightness profile to bacteria. The algorithm analyzes both darkfield and fluorescent images and rejects artifacts such as hot pixels, edge effects, bubbles, and large debris particles. The algorithm then determines if objects are stained by comparing the detected fluorescent objects and darkfield objects in an overlay. Fluorescent staining is reported for objects meeting a minimal peak signal in relation to the local background using the signal-to-noise ratio. Generally, objects must show a relative fluorescent signal at least 1.1-fold above the local background to be positive.

Table 3 shows the number of objects and relative percentage of objects detected *as E. coli* cells in the fluorescent image. FIG. 5 provides a graphical representation of the relative percentage of objects stained based on Table 3. The images in FIGS. 6A-1 to 6A-4 show darkfield images for the conditions while FIGS. 6B-1 to 6B-4 show the corresponding fluorescent images. The images revealed that unpurified samples were largely coated with residual blood debris and matrix materials and that the degree of debris burden was similar for the digested and undigested samples. Following purification by centrifugation, the undigested sample showed a reduced but significant amount of debris, some of which resulted in increased fluorescent background, somewhat limiting the ability to detect the stained *E. coli* cells. In contrast, the digested and purified sample was substantially free of debris and showed very clear, strong fluorescence of the *E. coli* cells.

In conclusion, this experiment demonstrates that protease digestion followed by a centrifugation step provided significant benefits to detection of bacteria in a slide-based FISH format.

**TABLE 3**

| *E. coli* slide stained with ECO/EUB probe cocktail. | | | | |
|---|---|---|---|---|
| **Purified** | **Protease** | **Total Objects** | **Total Cells Stained** | **Percent of Total Objects Stained** |
| No | Yes | 3326 | 579 | 17.4% |
| Yes | Yes | 234 | 212 | 90.6% |
| No | No | 4614 | 58 | 1.3% |
| Yes | No | 2312 | 153 | 6.6% |

### EXAMPLE 4

### Protease Treatment Dependent Bacterial Growth Inhibition in Rapid Blood Culture

Blood culture samples were treated with two different concentrations of protease to determine if protease used at a high concentration could produce inhibition of bacterial growth. Identification of a protease concentration dependent inhibitory effect on the growth rate of microbial cells is an important component of optimization of a rapid blood culture method. Growth of *E. coli* in simulated bacteremic blood cultures treated with a working protease concentration (0.2 mg/mL) and high protease concentration (20 mg/mL) was compared over a six (6) hour growth and digestion period.

### Methods

A mock blood culture specimen was created by addition of 10 mL of blood to a BioMerieux BacT/Alert Standard Aerobic bottle. A 1.0 mL portion of this specimen was used for each condition. Each sample was treated with saponin to a final concentration of 4 mg/mL and SPS to a final concentration of 0.96 mg/mL. Samples were then spiked with *E*. *coli* bacteria to a final concentration of 1x10² cfu/mL. The samples were treated with a Pronase solution containing either 0.2 mg/mL or 20 mg/mL. Samples were incubated at 35°C with agitation for 6 hours. A portion of each sample was removed for concentration determination at 2 hour time points during the incubation. The sampled bacteria were plated in 10-fold dilution series to determine the concentration of bacteria at each time point. Interval-by-interval growth rates, overall growth rate, and fold increase versus the starting concentration were determined using the calculated concentrations at each time point.

### Results

Table 4 shows the plate counts for the experiment along with the calculated concentrations for each sample at each time point. Table 5 shows the results of this experiment indicated that the *E. coli* bacteria showed a marked decrease in growth rate during each interval with the higher concentration of protease, resulting in approximately 8.5-fold lower overall increase during the 6 hour incubation, indicating that the protease was optimized for growth of bacteria at 0.2 mg/mL and that a higher concentration of protease can be detrimental to bacterial growth. FIG. 7 graphically presents the relative increase with the optimal and high protease concentrations, showing the inhibited growth with the high concentration.

In conclusion, optimal protease concentration must be used to obtain optimal growth rates within a given sample context.

**TABLE 4**

| Plating results and calculated concentrations of bacteria for each sample at the sampled time points. | | | | | | |
|---|---|---|---|---|---|---|
| **Time** | **Protease** | **Plate counts from 30 uL** | | | | **Conc (cfu/mL)** |
| | | **Undiluted** | **1/10** | **1/100** | **1/1000** | |
| 2 hr | Low | 7 | 0 | 0 | 0 | 2.3x10² |
| 2 hr | High | 8 | 0 | 0 | 0 | 2.7x10² |
| 4 hr | Low | TMTC* | 41 | 6 | 0 | 1.4x10⁴ |
| 4 hr | High | TMTC | 37 | 1 | 0 | 1.2x10⁴ |
| 6 hr | Low | TMTC | TMTC | TMTC | 67 | 2.2x10⁶ |
| 6 hr | High | TMTC | TMTC | 78 | 5 | 2.6x10⁵ |

| | | | | | | |
|---|---|---|---|---|---|---|
| *TMTC - too many to count | | | | | | |

**TABLE 5**

| Calculated growth rates for each growth interval, overall calculated growth rate over the 6 hour run, and calculated fold increase over the starting number of bacteria for each sample | | | | | |
|---|---|---|---|---|---|
| **Protease** | **0→2 Hours** | **2→4 Hours** | **4→6 Hours** | **Overall rate** | **Fold Growth** |
| Low | 0.60 | 2.96 | 3.65 | 2.40 | 22,000 |
| High | 0.70 | 2.74 | 2.22 | 1.89 | 2,600 |

### EXAMPLE 5

### Differential Protease Digestion and Detection of Live Microorganisms Compared to Antibiotic- and Ethanol-Killed Microorganisms

Certain assays for rapid detection of bacteria suffer from the presence of dead bacteria in the sample which results in false-positive detection that often has no clinical correlation with disease state. This can be the case when specimens are obtained from patients undergoing successful antibiotic therapy. A method for differentially detecting only live bacteria would be useful for reducing false-positive detection. The use of protease treatment of blood culture samples in a rapid blood culture format was investigated to determine whether protease treatment could facilitate differentiation of live microbial cells from dead microbial cells by eliminating false-positive detection of dead cells due to differential protease digestion of dead cells. The FISH staining assay was used to determine if dead cells could produce a false-positive signal and if the protease treatment would eliminate false-positive detection. As the FISH process employs ethanol fixation and heating, it is known to kill bacteria and thus, should be capable of detecting intact cells regardless of the viable state at the end of the incubation period.

### Methods

Bacteria of 4 different species were suspended in solution at high concentration (10⁷ cfu/mL) and then treated using the antibiotic gentamycin at 100 micrograms per mL for 1 hour to kill them. A parallel set of bacteria at the same concentration were treated with 80% ethanol to produce a killed set that was independent of antibiotic sensitivity. Finally, a third set of bacteria were not treated and served as live controls. Samples of each strain and each treatment were plated to determine the concentration of live bacteria. Aliquots of each preparation described above were spiked into tryptic soy broth medium or the same medium containing 0.2 mg/mL Pronase to obtain a suspension at the equivalent of 10⁵ cfu/mL. The samples were incubated at 35C for 4 hours. At the 1 hour point and at 4 hours a portion of each sample was removed for FISH analysis. Samples were centrifuged to exchange the buffer to electrokinetic concentration buffer. A portion of each sample was added to a flowcell of a microfluidic cassette having conductive top and slide surfaces. The cells were concentrated and immobilized to the poly-1-lysine coated slide surface by 1.5V applied for 5 minutes. The flowcells were washed with tris-buffered saline and the cells were permeabilized either by treatment with 80% ethanol 4 5 minutes (*E. coli, K. pneumoniae,* and *P. aeruginosa*) or lysozyme + lysostaphin treatment for 5 minutes followed by treatment with 80% ethanol for 5 minutes (S. aureus). Following permeabilization each flowcell was probed with a specific FISH probe as appropriate to the species of interest for 10 minutes in stringent conditions. Unbound probe was removed using 2-5 minute stringent washes and the staining was imaged to detect FISH signal.

Table 6 shows the plate count data and calculated concentrations from the run. These results indicated that the *E. coli, K. pneumoniae,* and *P. aeruginosa* were substantially killed by 1 hour in gentamycin but that a significant number of *S. aureus* remained alive in this condition. All strains treated with 80% ethanol showed no viable cells by plating. Calculated concentrations of live cells in the controls at T=0 were near 1x10⁵ cfu/mL for all strains. Table 7 presents the FISH assay data for the 1 hour time point and Table 8 shows the results for the 4 hour endpoint. A detection threshold of 1x10⁴ cells per mL was used as the threshold for a positive call in the FISH assay. At the 1 hour time point *E. coli* and *P*. *aeruginosa* treated with ethanol were not detected with the protease treatment but would have been called positive in the undigested condition. All gentamycin and live controls were detected regardless of the protease digest. At the later 4 hour time point, a much higher level of cells was detected in the live controls while none of the gentamycin or ethanol treated concentrations increased versus the 1 hour point. This result indicated that live cells detected by plating at T=0 may have died during the incubation period. Protease treatment removed false positive cells in gentamycin only for *P. aeruginosa* but decreased the ethanol treated false-positive in *E. coli* and *P. aeruginosa. K. pneumoniae* treated with gentamycin was negative for treated and untreated conditions, indicating that this treatment results in breakdown of the cell integrity without protease needed. *S. aureus* may require additional treatments to allow digest of dead cells to eliminate false-positive detection. Figures 8A, 8B, and 8C present graphically the results for *P. aeruginosa* detection using the FISH assay.

In conclusion, protease treatment can provide a means of differentiating live and dead organisms present in the sample when the analysis format does not provide sufficient discrimination of live and dead organisms. Alternative treatment methods may be needed for certain types of organisms to provide live-dead discrimination. *Results*

**TABLE 6**

| Plate count results and calculated concentrations for the various conditions | | | | | | |
|---|---|---|---|---|---|---|
| **Strain** | **Condition** | **Time** | **Dilution** | **Count** | **Conc (cfu/mL)** | **Amount Spiked (cfu)** |
| Ecol 35218 | GC | 0 hr | 1/1000 | 90 | 9x10⁵ | 9x10⁴ |
| Ecol 35218 | Gent | 0 hr | Undiluted | 1 | 10 | 1 |
| Ecol 35218 | EtOH | 0 hr | Undiluted | 0 | <10 | <1 |
| Kpne 13882 | GC | 0 hr | 1/1000 | 120 | 1x10⁶ | 1x10⁵ |
| Kpne 13882 | Gent | 0 hr | Undiluted | 6 | 60 | 6 |
| Kpne 13882 | EtOH | 0 hr | Undiluted | 0 | <10 | <1 |
| Paer 27853 | GC | 0 hr | 1/1000 | 84 | 8x10⁵ | 8x10⁴ |
| Paer 27853 | Gent | 0 hr | Undiluted | 2 | 20 | 2 |
| Paer 27853 | EtOH | 0 hr | Undiluted | 0 | <10 | <1 |
| Saur 12600 | GC | 0 hr | 1/1000 | 133 | 1x10⁶ | 1x10⁵ |
| Saur 12600 | Gent | 0 hr | Undiluted | 200 | 2x10³ | 200 |
| Saur 12600 | EtOH | 0 hr | Undiluted | 0 | <10 | <1 |

**TABLE 7**

| FISH results and calculated concentrations of cells resulting from the run at the 1 hour sampling point. | | | | | |
|---|---|---|---|---|---|
| **Strain** | **Condition** | **Protease** | **FISH Positive** | **Conc (cells/mL)** | **Positive Detection?** |
| Ecol 35218 | Control | Yes | 687 | 2.3x10⁶ | Yes |
| Ecol 35218 | Control | No | 689 | 2.3x10⁶ | Yes |
| Ecol 35218 | Gentamycin | Yes | 165 | 5.5x10⁵ | Yes |
| Ecol 35218 | Gentamycin | No | 263 | 8.8x10⁶ | Yes |
| Ecol 35218 | Ethanol | Yes | 1 | 3.3x10³ | No |
| Ecol 35218 | Ethanol | No | 13 | 4.3x10⁴ | Yes |
| Kpne 13882 | Control | Yes | 23 | 7.7x10⁴ | Yes |
| Kpne 13882 | Control | No | 54 | 1.8x10⁵ | Yes |
| Kpne 13882 | Gentamycin | Yes | 23 | 7.7x10⁴ | Yes |
| Kpne 13882 | Gentamycin | No | 52 | 1.7x10⁵ | Yes |
| Kpne 13882 | Ethanol | Yes | 204 | 6.8x10⁵ | Yes |
| Kpne 13882 | Ethanol | No | 147 | 4.9x10⁵ | Yes |
| Paer 27853 | Control | Yes | 27 | 9.0x10⁴ | Yes |
| Paer 27853 | Control | No | 80 | 2.7x10⁵ | Yes |
| Paer 27853 | Gentamycin | Yes | 16 | 5.3x10⁴ | Yes |
| Paer 27853 | Gentamycin | No | 111 | 3.7x10⁵ | Yes |
| Paer 27853 | Ethanol | Yes | 2 | 6.7x10³ | No |
| Paer 27853 | Ethanol | No | 90 | 3.0x10⁵ | Yes |
| Saur 12600 | Control | Yes | 488 | 1.6x10⁶ | Yes |
| Saur 12600 | Control | No | 270 | 9.0x10⁵ | Yes |
| Saur 12600 | Gentamycin | Yes | 32 | 1.1x10⁵ | Yes |
| Saur 12600 | Gentamycin | No | 42 | 1.4x10⁵ | Yes |
| Saur 12600 | Ethanol | Yes | 76 | 2.5x10⁵ | Yes |
| Saur 12600 | Ethanol | No | 102 | 3.4x10⁵ | Yes |

**TABLE 8**

| FISH results and calculated concentrations of cells at the 4 hour endpoint of the assay | | | | | |
|---|---|---|---|---|---|
| **Strain** | **Condition** | **Protease** | **FISH Stained Count** | **Conc (cells/mL)** | **Positive Detection?** |
| Ecol 35218 | Control | Yes | 5342 | 1.8x10⁷ | Yes |
| Ecol 35218 | Control | No | 4365 | 1.5x10⁷ | Yes |
| Ecol 35218 | Gentamycin | Yes | 136 | 4.5x10⁵ | Yes |
| Ecol 35218 | Gentamycin | No | 144 | 4.8x10⁵ | Yes |
| Ecol 35218 | Ethanol | Yes | 2 | 6.7x10³ | No |
| Ecol 35218 | Ethanol | No | 11 | 3.7x10⁴ | Yes |
| Kpne 13882 | Control | Yes | 1296 | 4.3x10⁶ | Yes |
| Kpne 13882 | Control | No | 1398 | 4.7x10⁶ | Yes |
| Kpne 13882 | Gentamycin | Yes | 5 | 1.7x10⁴ | Yes |
| Kpne 13882 | Gentamycin | No | 0 | <1x10³ | No |
| Kpne 13882 | Ethanol | Yes | 84 | 2.8x10⁵ | Yes |
| Kpne 13882 | Ethanol | No | 122 | 4.1x10⁵ | Yes |
| Paer 27853 | Control | Yes | 1758 | 5.9x10⁶ | Yes |
| Paer 27853 | Control | No | 3500 | 1.2x10⁷ | Yes |
| Paer 27853 | Gentamycin | Yes | 2 | 6.7x10³ | No |
| Paer 27853 | Gentamycin | No | 90 | 3.0x10⁵ | Yes |
| Paer 27853 | Ethanol | Yes | 0 | <1x10³ | No |
| Paer 27853 | Ethanol | No | 38 | 1.3x10⁵ | Yes |
| Saur 12600 | Control | Yes | 12505 | 4.2x10⁷ | Yes |
| Saur 12600 | Control | No | 9162 | 3.1x10⁷ | Yes |
| Saur 12600 | Gentamycin | Yes | 9 | 3.0x10⁴ | Yes |
| Saur 12600 | Gentamycin | No | 9 | 3.0x10⁴ | Yes |
| Saur 12600 | Ethanol | Yes | 35 | 1.2x10⁵ | Yes |
| Saur 12600 | Ethanol | No | 57 | 1.9x10⁵ | Yes |

### EXAMPLE 6

Table 9 provides examples of several classes of bacteria with examples of treatments that could be used to differentiate live and dead bacteria using a non-discriminate detection assay such as FISH as demonstrated in Example 5. This table suggests treatments that first permeabilize the bacteria, allowing the non-specific protease entry into the cell to complete the digest. For example, *S. aureus* is known to have a relatively impermeable coat made up of a thick peptidoglycan layer cross-linked with peptides resistant to many peptidases. The peptidase lysostaphin specifically cleaves this peptide structure and will permeabilize a *S*. *aureus* cell. It should be understood that the permeabilization conditions, including enzyme concentration and digest time, would need to be optimized to prevent permeabilization of live bacteria since these cells would be capable of some level of resistance to these enzymes. Since dead bacteria cannot remodel their coats, the optimized permeabilization condition would permanently open the inside of the cell allowing the protease or other enzyme access to the internal structures in order to degrade the target structures for the assay or release the contents of the dead cell, preventing detection. A combination of permeabilization and digesting agents may be needed to sufficiently remove dead cells while allowing detection of live cells using the endpoint assay.

**TABLE 9**

| Suggested treatments for discrimination of live and dead bacteria for certain classes | | | | | |
|---|---|---|---|---|---|
| **Bacteria Class** | **Example Species** | **Treatment for Permeabilization** | **Example(s)** | **Treatment for Digest** | **Example(s)** |
| Gram negative bacteria | *E. coli* | None | | Non-specific protease enzyme | Pronase Rhozyme Proteinase K |
| | *P. aeruginosa* | | | | |
| Staphylococcus | *S. aureus* | Specific peptidase enzyme | Lysostaphin | Non-specific protease enzyme | Pronase Rhozyme Proteinase K |
| Streptococcus, Lactococcus, Enterococcus | Enterococcus faecalis | Specific muramidase enzyme | Lysozyme | Non-specific protease enzyme | Pronase Rhozyme Proteinase K |

### EXAMPLE 7

Table 10 presents examples of different classes of bacteria that might require different growth intervals to reach detectable levels for a downstream assay. The table contemplates different concentrations of Pronase enzyme for used for different incubation periods based on the growth rate of the bacteria of interest. In addition, the expected concentrations of bacteria may differ based on the sample type and thus would require different treatment regimens and incubation periods to reach thresholds for detection. Adjustment of the enzyme concentration allows the appropriate level of digest for debris reduction without inhibiting bacterial growth. In the case of a slow growing *P. aeruginosa* (1.0 to 1.5 div/hour) starting with a moderate concentration of cells (100 to 1000 cfu/mL) such as might originate in a wound biopsy would require a moderate concentration of Pronase to reduce the debris over the 4 hours required for the bacteria to grow to a detectable concentration. The same species originating from a higher concentration sample such as a respiratory specimen may only require 1 hour in a higher enzyme concentration to digest debris without the requirement for growth. The specimen type and expected type of bacteria can be used to predict the conditions needed to obtain optimal debris reduction and growth to the detectable level.

**TABLE 10**

| Example conditions for growth and digest for different bacteria types. | | | | |
|---|---|---|---|---|
| **Bacteria Type** | **Typical Growth Rate (Div/hr)** | **Starting Bacteria Load** | **Enzyme Concentration (example for Pronase)** | **Incubation Time Required to Reach Threshold of 10⁴ cfu/mL** |
| Fast gram-negative bacteria (E. coli) | 2.0-2.5 | 1-10 cfu/mL | 0.02 mg/mL | 7 hours |
| | | 100 to 1000 cfu/mL | 0.2 mg/mL | 4 hours |
| | | >10,000 cfu/mL | 2 mg/mL | 1 hour |
| Slow gram-negative bacteria (P. aeruginosa) | 1.0-1.5 | 1-10 cfu/mL | 0.01 mg/mL | 14 hours |
| | | 100 to 1000 cfu/mL | 0.2 mg/mL | 4 hours |
| | | >10,000 cfu/mL | 2 mg/mL | 1 hour |
| Slow gram-positive bacteria | 0.5 to 1.0 | 1-10 cfu/mL | 0.005 mg/mL | 27 hours |
| | | 100 to 1000 cfu/mL | 0.01 mg/mL | 14 hours |
| | | >10,000 cfu/mL | 2 mg/mL | 1 hour |

It is believed that the disclosure set forth above encompasses at least one distinct invention with independent utility. While the invention has been disclosed in the exemplary forms, the specific embodiments thereof as disclosed and illustrated herein are not to be considered in a limiting sense as numerous variations are possible. Equivalent changes, modifications and variations of various embodiments, materials, compositions and methods may be made within the scope of the present disclosure, with substantially similar results. The subject matter of the inventions includes all novel and non-obvious combinations and subcombinations of the various elements, features, functions and/or properties disclosed herein and their equivalents.

The methods described herein may be implemented to facilitate rapid culturing and detection of microbial cells from samples. Benefits, other advantages, and solutions to problems have been described herein with regard to specific embodiments. However, the benefits, advantages, solutions to problems, and any element or combination of elements that may cause any benefit, advantage, or solution to occur or become more pronounced are not to be construed as critical, required, or essential features or elements of any or all the claims of the invention. Many changes and modifications within the scope of the instant invention may be made without departing from the spirit thereof, and the invention includes all such modifications. Corresponding structures, materials, acts, and equivalents of all elements in the claims below are intended to include any structure, material, or acts for performing the functions in combination with other claim elements as specifically claimed. The scope of the invention should be determined by the appended claims and their legal equivalents, rather than by the examples given above.

## Claims

1. A method comprising the steps of:
a) introducing a culture medium, a lytic agent and a cellular debris-cleaving enzyme to a blood sample comprising erythrocytes and at least one live microbial cell, wherein the introduction of the lytic agent and the cellular debris-cleaving enzyme to the blood sample degrades the erythrocytes to produce a concentration of free heme in the blood sample capable of depressing growth of the at least one live microbial cell, wherein the blood sample is a direct-from-patient blood culture or a positive blood culture;
b) introducing a heme polymerase to the blood sample;
c) incubating the blood sample with the lytic agent and the cellular debris-cleaving enzyme and the heme polymerase for a period of time to produce a digested blood sample;
d) producing growth of the at least one live microbial cell during the period of time to yield a threshold number of live microbial cells required for identification; and
e) determining the species and/or strain of at least one of the live microbial cells using automated digital microscopy.

2. The method of claim 1, wherein the cellular debris-cleaving enzyme comprises a hydrolase suitable to degrade blood sample debris.

3. The method of claim 2, wherein the hydrolase comprises a protease.

4. The method of claim 1, wherein the blood sample is polymicrobial.

5. The method of claim 1, wherein the automated digital microscopy comprises multiplexed automated digital microscopy.

6. The method of claim 1, further comprising introducing a portion of the digested blood sample to an automated digital microscopy system comprising a microscope, a pipetting robot, a multichannel fluidic cassette, and an analyzer to determine the identity of the at least one live microbial cell in the blood sample.

## Patentansprüche

1. Verfahren, umfassend die folgenden Schritte:
a) Einbringen eines Kulturmediums, eines lytischen Wirkstoffs und eines Zelltrümmer-spaltenden Enzyms in eine Blutprobe, die Erythrozyten und mindestens eine lebende mikrobielle Zelle umfasst, wobei das Einbringen des lytischen Wirkstoffs und des Zelltrümmer-spaltenden Enzyms in die Blutprobe die Erythrozyten abbaut, um eine Konzentration an freiem Häm in der Blutprobe zu erzeugen, die das Wachstum der mindestens einen lebenden mikrobiellen Zelle hemmen kann, wobei die Blutprobe eine direkt vom Patienten stammende Blutkultur oder eine positive Blutkultur ist;
b) Einbringen einer Häm-Polymerase in die Blutkultur;
c) Inkubieren der Blutprobe mit dem lytischen Wirkstoff und dem Zelltrümmer-spaltenden Enzym und der Häm-Polymerase für einen Zeitraum, um eine verdaute Blutprobe herzustellen;
d) Erzeugen eines Wachstums der mindestens einen lebenden mikrobiellen Zelle während des Zeitraums, um eine Schwellenanzahl von lebenden mikrobiellen Zellen zu ergeben, die zur Identifizierung erforderlich sind; und
e) Bestimmen der Art und/oder des Stammes von mindestens einer der lebenden mikrobiellen Zellen unter Verwendung automatisierter digitaler Mikroskopie.

2. Verfahren nach Anspruch 1, wobei das Zelltrümmer-spaltende Enzym eine Hydrolase umfasst, die geeignet ist, um Blutprobentrümmer abzubauen.

3. Verfahren nach Anspruch 2, wobei die Hydrolase eine Protease umfasst.

4. Verfahren nach Anspruch 1, wobei die Blutprobe polymikrobiell ist.

5. Verfahren nach Anspruch 1, wobei die automatisierte digitale Mikroskopie multiplexierte automatisierte digitale Mikroskopie umfasst.

6. Verfahren nach Anspruch 1, ferner umfassend das Einbringen eines Teils der verdauten Blutprobe in ein automatisiertes digitales Mikroskopiesystem, umfassend ein Mikroskop, einen Pipettierroboter, eine Mehrkanal-Fluidkassette und einen Analysator, um die Identität der mindestens einen lebenden mikrobiellen Zelle in der Blutprobe zu bestimmen.

## Revendications

1. Procédé comprenant les étapes consistant à:
a) introduire un milieu de culture, d'un agent lytique et d'une enzyme de séparation de débris cellulaires dans un échantillon de sang comprenant des érythrocytes et au moins une cellule microbienne vivante, dans lequel l'introduction de l'agent de lyse et de l'enzyme de séparation de débris cellulaires dans l'échantillon de sang dégrade les érythrocytes pour produire une concentration en hème libre dans l'échantillon de sang capable de ralentir la croissance d'au moins une cellule microbienne vivante, l'échantillon de sang étant une hémoculture directe du patient ou une hémoculture positive;
b) introduire une hème polymérase dans l'échantillon de sang;
c) incuber l'échantillon de sang avec l'agent lytique et l'enzyme de séparation de débris cellulaires et l'hème polymérase pendant une période de temps pour produire un échantillon de sang digéré;
d) produire une croissance d'au moins une cellule microbienne vivante pendant la période de temps nécessaire pour obtenir un nombre seuil de cellules microbiennes vivantes requises pour l'identification; et
e) déterminer l'espèce et/ou la souche d'au moins une des cellules microbiennes vivantes en utilisant une microscopie numérique automatisée.

2. Procédé selon la revendication 1, dans lequel l'enzyme de séparation de débris cellulaires comprend une hydrolase appropriée pour dégrader les débris d'échantillons de sang.

3. Procédé selon la revendication 2, dans lequel l'hydrolase comprend une protéase.

4. Procédé selon la revendication 1, dans lequel l'échantillon de sang est polymicrobien.

5. Procédé selon la revendication 1, dans lequel la microscopie numérique automatisée comprend une microscopie numérique automatisée multiplexée.

6. Procédé selon la revendication 1, comprenant en outre l'introduction d'une partie de l'échantillon de sang digéré dans un système de microscopie numérique automatisé comprenant un microscope, un robot de pipetage, une cassette fluidique à canaux multiples et un analyseur pour déterminer l'identité de la au moins une cellule microbienne vivante dans l'échantillon de sang.
